# EUROPEAN PATENT SPECIFICATION

(11) **EP 0 200 512 B1**
(45) Date of publication and mention of the grant of the patent: **25.05.1994**
(21) Application number: 86303189.4
(22) Date of filing: 28.04.1986
(51) Int. Cl.: C07K 7/06, C07K 7/24, A61K 37/02

(54) **Peptides and their use in therapy**
Peptide und deren Verwendung in der Therapie
Peptides et leur emploi en thérapie

(30) Priority: 30.04.1985 US 728847
(43) Date of publication of application: 05.11.1986
(73) Proprietor: THE ADMINISTRATORS OF THE TULANE UNIVERSITY EDUCATIONAL FUND, New Orleans, Louisiana 70112-2699 (US)
(72) Inventor: Coy, David H., New Orleans Louisiana 70115 (US); Moreau, Jacques-Pierre, Upton Massachusetts 01568 (US)
(74) Representative: Deans, Michael John Percy

(56) References cited:
- EP-A- 0 146 113
- FR-A- 2 328 453
- US-A- 3 856 770
- CHEMICAL ABSTRACTS, vol. 101, 1984, pages 287-288, abstract no. 3306u, Columbus, Ohio, US; M.H. ENGEL et al.: "Determination of the enantiomeric compostition of amine acid constituents of peptides with high performance liquid chromatography: a method for evaluating the effects of peptide sequence on the racemization rates of basle amine acids"
- CHEMICAL ABSTRACTS, vol. 103, 1985, page 68, abstract no. 98844w, Columbus, Ohio, US; V.J. HRUBY et al.: "Design and synthesis of peptide horones with prelonged activity at specific recepters"
- CHEMICAL ABSTRACTS, vol. 105, 1986, page 97, abstract no. 18672s, Columbus, Ohio, US; D.J. STAPLES et al.: "Carboxy-terminus of alpha-melanotropin: structural determinants important for melanotropic activity"
- CHEMICAL ABSTRACTS, vol. 100, 1984, pages 58-59, abstract no. 151154n, Columbus, Ohio, US; B.C. WILKES et al.: "Differentiation of the structural features of melanotropins important for biological potency and prolonged activity in vitre"
- CHEMICAL ABSTRACTS, vol. 94, 1981, page 94, abstract no. 25415t, Columbus, Ohio, US; J.M. STEWART et al.: "Functional and evolutionary crossover in peptide hormones: LRH and ACTH"
- Eur. J. Pharm., 76 (1981), 73-79
- Eur. J. Pharm., 92 (1983), 231-236

## Description

This invention relates to therapeutic peptides.

ACTH-like neuropeptides can facilitate nerve regeneration in the central and peripheral nervous systems. Bijlsma et al. Eur. J. Pharm., 76 (1981) 73-79 and Bijlsma et al. Eur. J. Pharm., 92 (1983) 231-236 describe using ACTH-like neuropeptides, including H-Met(O₂)-Glu-His-Phe-D-Lys-Phe-OH and α-MSH, to restore sensorimotor function in rats having crushed sciatic nerves (where no D- or L-isomeric designation is given herein, the naturally occurring L-isomer is intended).

In general, the invention features a peptide of the formula:

A¹-D-Ala-A³-A⁴-Phe-Arg-Trp-Gly-NH₂,

wherein
- A¹: is H or acetyl;
- A³: is Glu or Gln; and
- A⁴: is His or Tyr;
or a pharmaceutically acceptable salt thereof.

In a preferred example of the peptide A¹ is H, A³ is Gln and A⁴ is Tyr.

As we explain below, a therapeutically effective amount of the peptide and a pharmaceutically acceptable carrier substance, e.g. magnesium carbonate or lactose, together form a therapeutic composition for aiding in regenerating nerves of the peripheral nervous system and the brain. The composition can be in the form of a pill, tablet, capsule, liquid, or sustained release tablet for oral administration; a liquid for nasal administration; or a liquid for intravenous, subcutaneous, parenteral, or intra-peritoneal administration.

Other features and advantages will be apparent from the following description of preferred examples.

We now describe the structure, synthesis, and use of preferred examples.

The peptides described herein have the general formula.

A¹-D-Ala-A³-A⁴-Phe-Arg-Trp-Gly-NH₂,

wherein
- A¹: is H or acetyl;
- A³: is Glu or Gln; and
- A⁴: is His or Tyr.
They all have an HN₂ at the carboxy terminal end, in addition to Phe at position 4, Arg at position 5, and Trp at position 6.

The peptides can be provided in the form of pharmaceutically acceptable salts. Examples of preferred salts are those of therapeutically acceptable organic acids, e.g., acetic, lactic, maleic, citric, malic, ascorbic, succinic, benzoic, salicylic, methanesulphonic, toluenesulphonic, or pamoic acid, as well as polymeric acids such as tannic acid or carboxymethyl cellulose, and salts with inorganic acids such as hydrohalic acids, e.g., hydrochloric acid, sulphuric acid, or phosphoric acid.

The synthesis of H-D-Ala-Gln-Tyr-Phe-Arg-Trp-Gly-NH₂ follows.

Other peptides can be prepared by making appropriate modifications of the following synthetic method.

The first step is the preparation of D-Ala-Gln-Tyr -Phe-Arg-Trp-Gly-benzhydrylamine-resin, as follows.

Benzyhydrylamine-polystyrene resin (Bachem, Inc.) (1.00 g, 0.5 mmole) in the chloride ion form is placed in the reaction vessel of a Beckman 990B peptide synthesizer programmed to perform the following reaction cycle: (a) CH₂Cl₂; (b) 33% trifluoroacetic acid CH₂Cl₂ (2 times for 1 and 25 min. each); (c) CH₂Cl₂; (d) ethanol; (e) CH₂Cl₂; (f) 10% triethylamine in CHCl₃; and (g) CH₂Cl₂.

The neutralized resin is stirred with Boc-glycine and diisopropylcarbodiimide (1.5 mmole) in CH₂Cl₂ for 1 hour and the resulting amino acid resin is then cycled through steps (a) to (g) in the above wash program. The following amino acids (1.5 mmole) are then coupled successively by the same procedure: Boc-Trp, Boc-tosyl-Arg, Boc-Phe, Boc-carbenzoxy-His, Boc-Gln, and Boc-D-Ala.

From the above resin is prepared H-D-Ala-Gln-Tyr -Phe-Arg-Trp-Gly-NH₂, as follows.

A mixture of the above heptapeptide resin,
(0.5 mmole) and a solution of 4 ml anisole, and 36 ml hydrogen fluoride is stirred at 0°C for 45 minutes. Excess hydrogen fluoride is evaporated rapidly under a stream of dry nitrogen, after which the free peptide is precipitated and washed with ether.

The peptide is then dissolved in a minimum volume of 50% acetic acid and eluted on a column (2.5 x 100 mm) of Sephadex G-25. Fractions containing a major component, as determined by u.v. absorption and thin layer chromatography (tlc) are pooled and evaporated to a small volume in vacuo. This solution is applied to a column (2.5 x 50 cm) of octadecylsilane-silica (Whatman LRP-1, 15-20 µ m mesh size) which is eluted with a linear gradient of 0-40% acetonitrile in 20% acetic acid in water. Fractions are examined by tlc and analytical high performance liquid chromatography (hplc) and pooled to give maximum purity. Repeated lyophilization of the solution from water gives the product as a white, fluffy powder.

This material is found to be homogeneous by hplc and tlc. Amino acid analysis of an acid hydrolysate confirms the composition of the peptide.

When administered to a mammal (e.g., orally, intravenously, parenterally, nasally, or by suppository), the peptides are effective in aiding in regenerating nerves of the peripheral and central nervous systems following nerve damage. The peptides are administered beginning directly following the injury, for a period of ten days or more. Administration is daily or every other day.

The peptides can be used to treat nerve crush lesions and neuropathies of alcoholic, diabetic, or toxic substance exposure origins. The peptides can also be used to aid in suturing severed nerves. The peptides can promote the growth of new nerve processes, as well as enhance the connection of nerves to muscles.

The peptides can be administered to a patient in a dosage of 1 mcg/kg/day to 250 mcg/kg/day, preferably 5-100 mcg/kg/day.

## Claims

1. A peptide of the formula:
A¹-D-Ala-A³-A⁴-Phe-Arg-Trp-Gly-NH₂,
wherein
A¹ is H or acetyl;
A³ is Glu or Gln; and
A⁴ is His or Tyr;
or a pharmaceutically acceptable salt thereof.

2. A peptide according to Claim 1 characterised in that, A¹ is H; A³ is Gln; and A⁴ is Tyr; or a pharmaceutically acceptable salt thereof.

3. A therapeutic composition comprising a therapeutically effective amount of a peptide according to any preceding claim together with a pharmaceutically acceptable carrier substance.

4. A composition according to Claim 3, in the form of a pill, tablet, capsule, liquid or sustained release tablet for oral administration; in the form of a suppository; in the form of a liquid capable of being administered nasally; or in the form of a liquid capable of being administered intravenously, subcutaneously, parenterally, or intraperitoneally.

5. A peptide according to Claim 1 or Claim 2, for use in therapy in aiding the regeneration of nerves, in the treatment of nerve crush lesions, in the treatment of neuropathies of alcoholic, diabetic, or toxic substance exposure origins, in aiding the suturing of several nerves, in promoting the growth of new nerve processes, or in enhancing the connection of nerves to muscles.

6. A method of synthesizing a peptide of the formula:
A¹-D-Ala-A³-A⁴-Phe-Arg-Trp-Gly-NH₂,
wherein
A¹ is H or acetyl;
A³ is Glu or Gln; and
A⁴ is His or Tyr;
the method comprising the steps of providing Gly-NH₂ protected with a protecting group, reacting said protected Gly-NH₂ with resin to form a resin-bound protected Gly-NH₂, washing said resin-bound protected Gly-NH₂, adding successively to said resin-bound protected Gly-NH₂ protected Trp, protected Arg, protected Phe, protected A⁴, protected A³ and protected D-Ala, acetylating or hydrogenating the resulting product, and treating said product to remove said resin.

## Patentansprüche

1. Ein Peptid der Formel:
A¹-D-Ala-A³-A⁴-Phe-Arg-Trp-Gly-NH₂,
worin
A¹ H oder Acetyl ist ;
A³ Glu oder Gln ist ; und
A⁴ His oder Tyr ist ;
oder ein pharmazeutisch annehmbares Salz davon.

2. Ein Peptid nach Anspruch 1, dadurch gekennzeichnet, daß A¹ H ist ; A³ Gln ist ; und A⁴ Tyr ist ; oder ein pharmazeutisch annehmbares Salz davon.

3. Eine therapeutische Zusammensetzung, umfassend eine therapeutisch wirksame Menge eines Peptids nach einem vorhergehenden Anspruch zusammen mit einer pharmazeutisch annehmbaren Trägersubstanz.

4. Eine Zusammensetzung nach Anspruch 3 in Form einer Pille, Tablette, Kapsel, Flüssigkeit oder Depot-Tablette zur oralen Verabreichung, in Form eines Zäpfchens, in Form einer Flüssigkeit, die nasal verabreichbar ist, oder in Form einer Flüssigkeit, die intravenös, subkutan, parenteral oder intraperitoneal verabreichbar ist.

5. Ein Peptid nach Anspruch 1 oder Anspruch 2 zur Verwendung in der Therapie zur Unterstützung der Regeneration von Nerven, in der Behandlung traumatischer Nervenläsionen, in der Behandlung von Neuropathien, die durch Alkohol, Diabetes oder Einwirkung toxischer Substanz verursacht sind, zur Unterstützung des Nähens verschiedener Nerven, zur Förderung des Wachstums neuer Nervenfortsätze oder zur Verbesserung der Verbindung von Nerven an Muskeln.

6. Ein Verfahren zur Synthese eines Peptids der Formel :
A¹-D-Ala-A³-A⁴-Phe-Arg-Trp-Gly-NH₂,
worin
A¹ H oder Acetyl ist ;
A³ Glu oder Gln ist ; und
A⁴ His oder Tyr ist ;
wobei das Verfahren die Schritte umfaßt, mit einer Schutzgruppe geschütztes Gly-NH₂ bereitzustellen, das geschützte Gly-NH₂ mit Harz umzusetzen, um ein harzgebundenes geschütztes Gly-NH₂ zu bilden, das harzgebunde geschützte Gly-NH₂ zu waschen, nacheinander zu dem harzgebunden geschützten Gly-NH₂ geschütztes Trp, geschütztes Arg, geschütztes Phe, geschütztes A⁴, geschütztes A³ und geschütztes D-Ala hinzufügen, das resultierende Produkt zu acetylieren oder zu hydrieren und das Produkt zur Entfernung des Harzes zu behandeln.

## Revendications

1. Un peptide de formule :
A¹-D-Ala-A³-A⁴-Phe-Arg-Trp-Gly-NH₂,
dans laquelle
A¹ est H ou le groupe acétyle ;
A³ est Glu ou Gln ; et
A⁴ est His ou Tyr ;
ou un sel pharmaceutiquement acceptable de ce dernier.

2. Un peptide selon la revendication 1, caractérisé par le fait que A¹ est H ; A³ est Gln ; et A⁴ est Tyr ; ou un sel pharmaceutiquement acceptable de ce dernier.

3. Une composition thérapeutique comprenant une quantité thérapeutiquement efficace d'un peptide selon l'une quelconque des revendications précédentes avec une substance-support pharmaceutiquement acceptable.

4. Une composition selon la revendication 3, sous la forme d'une pilule, d'un comprimé, d'une capsule, d'un liquide ou d'un comprimé à libération entretenue pour administration par voie orale ; sous la forme d'un suppositoire ; sous la forme d'un liquide pouvant être administré par voie nasale ; ou sous la forme d'un liquide pouvant être administré par voies intraveineuse, sous-cutanée, parentérale ou intrapéritonéale.

5. Un peptide selon la revendication 1 ou 2, destiné à être utilisé en thérapie pour faciliter la régénération de nerfs, dans le traitement de lésions accompagnées de l'écrasement de nerfs, dans le traitement de neuropathies d'origines alcoolique, diabétique ou dues à une exposition à une substance toxique, pour faciliter la suture de plusieurs nerfs, pour promouvoir le développement de nouveaux processus nerveux ou pour consolider la connexion de nerfs à des muscles.

6. Une méthode de synthèse d'un peptide de formule :
A¹-D-Ala-A³-A⁴-Phe-Arg-Trp-Gly-NH₂,
dans laquelle
A¹ est H ou le groupe acétyle ;
A³ est Glu ou Gln ; et
A⁴ est His ou Tyr ;
cette méthode comprenant les phases consistant à préparer Gly-NH₂ protégé par un groupe protecteur ; à faire réagir ledit Gly-NH₂ protégé, avec une résine, pour former Gly-NH₂ protégé, lié à une résine ; à laver ledit Gly-NH₂ protégé lié à une résine ; à ajouter successivement audit Gly-NH₂ protégé lié à une résine, Trp protégé, Arg protégé, Phe protégé, A⁴ protégé, A³ protégé et D-Ala protégé ; à acétyler ou à hydrogéner le produit résultant et à traiter ledit produit pour éliminer ladite résine.
